# EUROPEAN PATENT SPECIFICATION

(11) **EP 4 329 593 B1**
(45) Date of publication and mention of the grant of the patent: **18.02.2026**
(21) Application number: 22724985.1
(22) Date of filing: 28.04.2022
(51) Int. Cl.: A61B 5/00, A61B 5/11, A63B 71/00, A63B 71/08, G01P 15/08

(54) **IMPACTS MONITORING SCREENING OUT FALSE POSITIVE RESULTS**
SCHOCKÜBERWACHUNG, DIE FALSCH-POSITIVE ERGEBNISSE AUSSCHLIESST
MONITORAGE D'IMPACTS EXCLUANT LES FAUX-POSITIFS

(30) Priority: 29.04.2021 US 202163181574 P
(43) Date of publication of application: 06.03.2024
(73) Proprietor: Prevent Biometrics, Inc., Edina, MN 55435 (US)
(72) Inventor: BARTSCH, Adam, Edina, Minnesota 55435 (US); THIBADO, Jason, Edina, Minnesota 55435 (US); GOODGER, Drew, Edina, Minnesota 55435 (US)
(74) Representative: Murgitroyd & Company
(86) International application number: PCT/US2022/026647
(87) International publication number: WO 2022/232344

(56) References cited:
- US-A1- 2012 210 498
- US-A1- 2014 257 051

## Description

### TECHNOLOGICAL FIELD

The present disclosure relates to systems for impact monitoring. More particularly, the present disclosure relates to systems for accurately sensing impacts to the human head or body and accounting for and/or screening out false positive results. Still more particularly, the present disclosure relates to using multiple proximity sensor readings to rule out false positives and, further, to rely on true positive sensor readings to monitor athlete workload.

### BACKGROUND

The background description provided herein is for the purpose of generally presenting the context of the disclosure. Work of the presently named inventors, to the extent it is described in this background section, as well as aspects of the description that may not otherwise qualify as prior art at the time of filing, are neither expressly nor impliedly admitted as prior art against the present disclosure.

Sensing head impacts for purposes of assessing risk of brain damage has come to the forefront in many activities. Sensor systems on helmets, on skin patches, on mouth guards, or on other systems or devices have been studied and implemented. Several difficulties exist with respect to obtaining accurate and precise results. For example, helmets are designed to reduce and/or distribute impact loads to the head via a relatively loose helmet-to-head coupling, so sensors on the helmet may sense impacts that are higher or otherwise different than those that are passed onto the head and the direction and/or magnitude of the impact on the helmet may create uncertainty as to the forces experienced by the head. One particular difficulty with respect to obtaining accurate and precise results across many systems relates to false positives. For example, impacts may be sensed by equipment when a user drops the equipment or drops or sets down a bag that the equipment is in. Still other impacts may be sensed when a bag is being carried and swings against an obstruction. These and other impacts that may be sensed by an impact sensor are not relevant to head impacts and, preferably, would be screened out of the data that is collected and more seriously assessed.

Some preliminary efforts to rule out false positives for mouthguard-based systems have focused on assuring that the mouth guard is in on the teeth. For example, a proximity sensor has been suggested as a method for determining when the mouthguard is on the teeth. However, when not in use, users have been known to turn the mouthguard sideways and chew on it, which may trigger the proximity sensor(s) and result in false positive readings. Also, a user may put a finger, lip, or article of clothing in front of the sensor causing the system to believe, so to speak, that it is on the teeth. Simple on/off switches have also been suggested, but may only be helpful to the extent the device is turned off when not being actively used during situations where impact results are desired. (i.e., during a game when the player is not on the field and has his/her helmet off or mouthguard out of the mouth). Counting on users to constantly activate and deactivate sensors is not reliable.

US2014/257051 A1 concerns a device for detecting on-body impacts. The device is placed on a human subject to detect impacts on the human subject. The device includes a base member, one or more engagement sensors to detect whether the device is properly placed on the human subject, and one or more motion sensors to detect the kinematics of the human subject. The device also includes a processing unit that includes methodology to detect false positives such as chewing, dropping, and throwing.

US2012/210498 A1 describes a protective headgear position and impact sensor for use with hard hats, helmets, or other headgear. Proximity sensors are used to detect whether the headgear is being worn by the user. Additional features may determine the nature of a head impact and store data related to wear of the headgear by the user. Yet other features may allow the headgear to serve as a security device, allowing entry into a facility only if the headgear is in position and if it is properly associated with an authorized individual who is wearing the headgear.

### SUMMARY

The invention is a system and a method as defined in the appended claims.

The following presents a simplified summary of one or more embodiments of the present disclosure in order to provide a basic understanding of such embodiments. This summary is not an extensive overview of all contemplated embodiments, and is intended to neither identify key or critical elements of all embodiments, nor delineate the scope of any or all embodiments.

According to the invention, the system for sensing true positive impacts includes a sensing device configured for secured coupling to a user. The sensing device includes a sensor configured for sensing accelerations associated with an impact event and for generating a signal based on the impact event. The sensing device also includes a control sensor for sensing when the sensing device is in position for sensing. The sensing device also includes a computer-readable storage medium having instructions stored thereon for receiving and capturing the signal from the sensor and comparing first and second signals from the control sensor to determine if the signal is a true positive signal, the first signal from the control sensor being captured at a time prior to the impact event or during an early portion of the impact event and the second signal from the control sensor being captured at a later portion of the impact event or after the impact event. The sensing device also includes a processor for processing the instructions to capture the signal, perform the comparing, and identify the signal as a true positive signal indicative of an impact of the user based on the comparing.

According to the invention, the method of isolating true positive impacts and storing the true positive impacts includes sensing and recording a signal of an impact event from a motion sensor. The method also includes receiving a first control signal from a control sensor prior to the impact event and receiving a second control signal from the control sensor after the impact event. The method also includes comparing the first control signal to the second control signal. The method also includes identifying the signal of an impact event as a true positive signal based on the comparing and discarding the impact signal if the impact signal is not identified as a true positive.

While multiple embodiments are disclosed, still other embodiments of the present disclosure will become apparent to those skilled in the art from the following detailed description, which shows and describes illustrative embodiments of the invention. As will be realized, the various embodiments of the present disclosure are capable of modifications in various obvious aspects, all without departing from the scope of the present disclosure. Accordingly, the drawings and detailed description are to be regarded as illustrative in nature and not restrictive.

### BRIEF DESCRIPTION OF THE DRAWINGS

While the specification concludes with claims particularly pointing out and distinctly claiming the subject matter that is regarded as forming the various embodiments of the present disclosure, it is believed that the invention will be better understood from the following description taken in conjunction with the accompanying Figures, in which:
FIG. 1 is a perspective view of athletes wearing an impact sensor, according to one or more embodiments, and experiencing bodily impacts while participating in an athletic event.
FIG. 2 is a diagram of a motion sensing device in communication with an outside computing device via one or more communication systems, according to one or more embodiments.
FIG. 3 is a diagram depicting a method of sensing true positive impacts, according to one or more embodiments.
FIG. 4 is a diagram depicting a method of workload monitoring, according to one or more embodiments.
FIG. 5 is a diagram depicting average workloads for several athletes, according to one or more embodiments.
FIG. 6 is a screen shot of a workload interface, according to one or more embodiments.
FIG. 7 is a diagram depicting proximity sensor results over time.

### DETAILED DESCRIPTION

The present application, in one or more embodiments, relates to impact sensing and, in particular, to a method for ruling out false positive readings on impact sensors. The sensors may be used for sensing impacts to athletes, military personnel, and/or other users. The ability to rule out false positives may allow for a better ability to focus on meaningful impact data and develop and/or generate meaningful protocols or other systems for identifying injury-inducing impacts or a series of impacts that collectively can cause injury. Moreover, the ability to identify relevant impact data amidst an array of otherwise comingled data may allow for further uses beyond injuries that are caused by impacts. For example, in one or more embodiments, a method of assessing the workload of an athlete may be provided. This method may be advantageous for assessing the performance and/or exertion level of athletes that may not have readily available or measurable analytic data. For example, defensive and offensive linemen in football may not move down field much and, as such, may not be commonly be assessed by speed or distance covered, range, etc. (e.g., as compared to running backs and wide receivers that are commonly assessed by speed). Moreover, how hard linemen are working or being worked on any given day may be difficult to assess. The present system may provide a method for assessing this information, which may allow for a better assessment metric and the opportunity for a safer practice session or sessions.

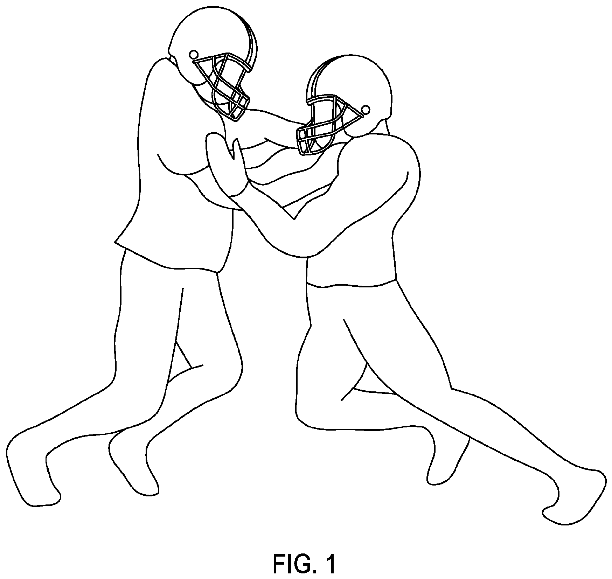

As shown in FIG. 1, two linemen are engaged with one another in a pushing match commonly occurring throughout a football game. The offensive lineman may be protecting the quarterback, for example, and/or attempting to create a hole for a running back to run through. The defensive lineman may be attempting to get passed the offensive lineman to tackle or, otherwise, interfere with the quarterback or the defensive lineman may be attempting to reach and tackle a running back if the ball has been handed off. Throughout this pushing and shoving exchange, multiple impacts may be sensed by an impact sensor on, for example, a mouthguard of one or both linemen. As discussed in more detail below, the impacts sensed by the impact sensor may be helpful in assessing workload of either or both linemen.

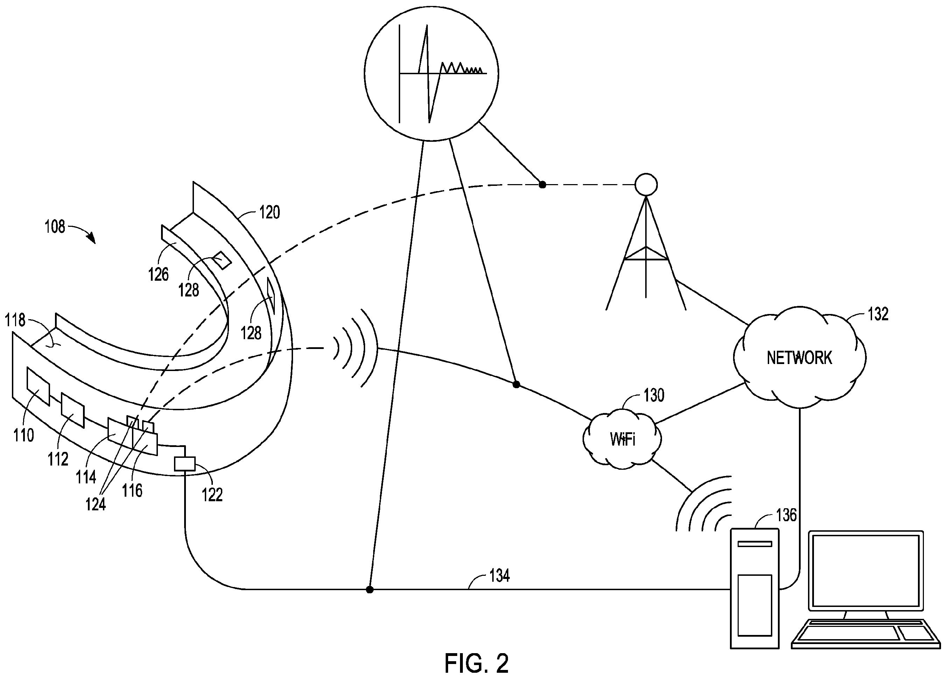

Referring now to FIG. 2, a motion sensing device 108 worn by one or both linemen is shown. The sensing device may be adapted to sense impacts, store and/or analyze the impacts, and transmit the impact data to one or more additional devices. In one or more embodiments, the sensing device may be in the form of a mouthguard configured for kinematic sensing such as a mouthguard worn by athletes during athletic events and having sensing equipment thereon for sensing head impacts. In one or more embodiments, the sensing device 108 may include a body portion and an electronic system including a power source 110, one or more sensors 112, a data storage medium 114, a processor 116, input/output devices 122, and/or receiving and transmitting systems 124.

The body portion may be in the form of a mouthguard or an alternative wearable device may be used. The alternative wearable device may be, for example, a body or skin patch, a clothing patch, a head band, mouthpiece, earpiece, or other wearable device. In the case of a mouthguard, the mouthguard may include a dentition portion 118, a labial portion 120, and a lingual portion 126. The dentition portion 118 may be generally flat and u-shaped and adapted for resting on and/or being positioned between the crown of the teeth. In one or more embodiments, the dentition portion 118 may be adapted for molding to the teeth using a heating and biting process or the dentition portion may be custom fitted and molded, for example. The dentition portion may include an inner u-shaped edge and an outer u-shaped edge. The labial portion120 may extend upwardly and/or downwardly from the outer u-shaped edge of the dentition portion and may be configured to protect the labial surface of the upper and/or lower teeth. The lingual portion 126 may be provided extending upward and/or downward from the inner u-shaped edge of the dentition portion and may be configured to keep the tongue from slipping between the teeth, for example.

The electronic system may be arranged on the surface of, lodged within, molded within, or otherwise associated with the body portion. In one or more embodiments, the electronics system may be over-molded within the labial or lingual portion of the mouthguard. In one or more embodiments, the mouthguard may be manufactured consistent with the system and methods described in U.S. Patent Application No.: 16/682,656, filed on November 13, 2019, and entitled Impact Sensing Mouthguard. Still other approaches to manufacturing the mouthguard may be used.

The power source 110 may be an electric power source configured for providing power to the sensors, the storage medium, and the processor. In one or more embodiments, the power source may be in the form of a battery such as a nickel cadmium alloy battery, a metal hydride battery, microscopic batteries, or another battery suitable for powering micro electro-mechanical devices.

The sensors 112 may include sensors adapted for sensing kinematic body motion of an athlete, military personnel, or other human user such as those resulting from bodily impact or collision, for example. In one or more embodiments, the sensors may include accelerometers including linear accelerometers, angular accelerometers, gyroscopes, or other motion sensing micro electro-mechanical devices. In one particular embodiment, a 3-axis linear accelerometer may be provided together with a 3-axis gyroscope. The linear accelerometer may be configured for sensing linear accelerations along x, y, and z axes and the gyroscope may be configured for sensing angular velocities along the same set of x, y, and z axes. In one or more embodiments, manufacturing techniques may be used to align the local axes of the two separate sensors. In other embodiments, mathematical techniques may be used to determine any out of alignment issues and to normalize the two sets of data to reflect the same set of axes. In one or more embodiments, the normalization may be performed to correspond with a human anatomy axis, where X may be directed anteriorly, Y may be directed laterally, and Z may be directed upward.

As mentioned, the sensor 112 may be kinematic sensors. That is, the sensors 112 may be adapted for sensing kinematic motion of the human body. As such, the sensors may be designed, sized, and calibrated for sensing a range of accelerations commonly reflected by the motion of an athlete during athletic events and, in particular, during an impact event. For example, the sensors may be calibrated for sensing accelerations having magnitudes ranging from approximately 0 g's to approximately 300 g's, or from approximately 0 g's to approximately 250 g's, or from approximately 0 g's to 200 g's. Moreover, the sensors may have sample frequencies adapted for modeling motions of the human body in response to impacts. Impacts to the human body such as those experienced by football players or other athletes may occur over a period of time of approximately 10 milliseconds. In one or more embodiments, the accelerometers, gyroscopes, and other MEMs sensing devices of the present disclosure may have sample rates ranging from approximately 10 Hz to approximately 10,000 Hz, or from approximately 500 Hz to approximately 7500 Hz, or from approximately 1000 Hz to approximately 5000 Hz, or from approximately 1500 Hz to approximately 4500 Hz, or from approximately 2500 Hz to approximately 4000 Hz, or from approximately 3000 Hz to approximately 3500 Hz, or a sample rate of approximately 3200 Hz may be used. In one or more embodiments, an accelerometer such as an ADXL 372 manufactured by Analog Devices may be provided. This particular accelerometer may have a range of 200 g's and a bandwidth or sample rate of 3200 Hz.

Apart from the kinematic sensors, the one or more sensors may include control sensors 128 adapted for use to filter data and/or control the kinematic sensors 112 to avoid collecting data, for example. In one or more embodiments, the control sensors 128 may be proximity sensors, capacitive sensors or other sensors allowing for assessments to be made about whether the mouthguard is actually in the mouth and/or on the teeth of the user. This information can be used to awaken and/or trigger the electronics of the system, to filter out data if the system is sensing information when the device is not on the teeth and/or for other purposes.

In one or more embodiments, a control sensor may be arranged facing inward from the labial portion. As such, unless an object is within the channel formed by the labial and lingual flange 120/126 and the dentition portion 118, the system may be in a sleep or off state or data collected during that timeframe may be ignored. That is, when the mouthguard is on the teeth, the sensors may be covered and an object may be sensed within the channel, so sensing with the kinematic sensors may be appropriate. However, when the mouthguard is in a case or in a gym or military bag, the sensing may not be appropriate and it may also be unlikely that the sensors would be closely covered in those situations. In some cases, users may accidentally or intentionally trigger the control sensor by obstructing the sensor. For example, a user may obstruct the sensor with their finger or the user may pop the mouthguard off of their teeth and cover the sensor with their tongue or chew on the mouthguard causing the u-shaped channel to collapse and triggering the sensor. For purposes of addressing these situations where impacts sensed during these situations may be false positives, multiple sensors have been proposed. However, the present disclosure proposes a method of use that may lessen the need for two sensors by using a single control sensor in a different manner as discussed in more detail below. Nonetheless, multiple control sensors may still be provided for situations where users are, for example, missing teeth or have mouthguards or systems that line up with gums instead of teeth, or have other anatomical issues that make covering one sensor in a particular location difficult.

The data storage medium 114 of the electronic system may be a computer readable data storage medium such as volatile memory (e.g., random access memory (RAM)) and/or non-volatile memory (e.g., read-only memory (ROM, EPROM, EEPROM, etc.)). A basic input/output system (BIOS) can be stored in the non-volatile memory (e.g., ROM), and may include basic routines facilitating communication of data and signals between components within the system. The volatile memory may additionally include a high-speed RAM, such as static RAM for caching data. In addition to facilitating communication and data and signals, the memory may include computer readable instructions particularly adapted for communicating with separate computing systems (e.g., for performing receiving and transmitting operations), controlling on/off states of the sensors, for monitoring the condition of the mouthguard (e.g., on the teeth, off the teeth, etc.), for receiving sensor data, for controlling on/off and/or sleep states of the processors, etc. In one or more embodiments, the memory may include computer readable instructions adapted to receive, store, and and/or analyze sensor data such as accelerometer signals received from a head impact and/or a blast event. These computer-readable instructions are discussed in more detail below.

The computer processor 116 of the electronic system may be adapted to execute the computer-readable instructions on the data storage medium. For example, the various sets of instructions on the computer readable storage medium for facilitating communication between components within the system, the more specific controls of the sensors and the receipt of data from the sensors may all be processed and/or executed by the processor. In one or more embodiments, the processor may be a high performance unit such as a 32-bit microcontroller from ST Microelectronics, for example.

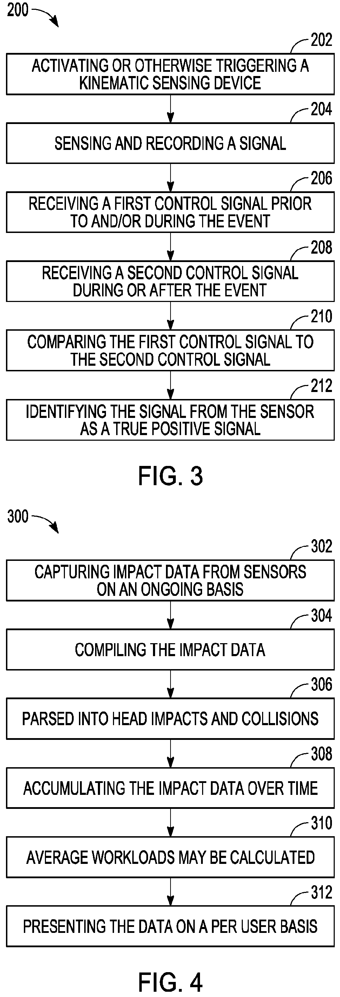

Input/output devices 122 may also be present on the sensing device for powering up, for example, resetting, or otherwise directly interacting with the sensing device. Moreover, while the sensing device has been said to have computer-readable instructions and a processor for analyzing the sensor data or sensor signals, this analysis may be performed by a separate computing system as well. As such, the sensing device may be equipped with receiving and transmitting systems 124 operable by the processor and the storage medium to receive instructions from outside computing devices and/or to transmit information including the sensor data to outside computing devices. The receiving and transmitting devices may include local area network (LAN) type devices and may include WiFi, Bluetooth, Zigbee, or other relatively local area communication systems. Alternatively or additionally, the receiving and transmitting devices may include wide area network (WAN) communication capabilities such as cellular or other communication systems. As shown in FIG. 3, sensor data may be transmitted via a local area network 130, a wide area network 132 such as the internet, or via a direct hardwire communication 134 to an outside computing device 136 for monitoring and/or analysis. In one or more embodiments, a combination of these communication systems may be used.

The sensing device 108 may be the same or similar to those that are shown and described in U.S. Patents 9,044,198, 9,149,227, 9,289,176, and 9,585,619. Still other sensing devices and process may be used, such as those described in U.S. Patents 8,537,017, 8,466,794, 9,526,289, 8,554,495, and 9,554,607. Still other sensing systems and processes may be used, such as those described in U.S. Patent Applications 13/009,580, 14/040,157, and 14/040,111.

In operation and use, the sensing device may be used to monitor and/or analyze impacts to athletes, military personnel, or other users. In particular, and as shown in FIG. 3, a method of sensing true positive impacts (200) may be provided. The method is focused on ruling out false positive sensor data allowing for a focus on true positive sensor data. In particular, the method may include a unique way of determining when a sensor is positioned on the teeth of a user during an impact. That is, when an impact sensing mouthguard is on the teeth of a user, it may be considered to be in position for sensing and impacts sensed when the mouthguard is in this position may be considered to be true positive sensor results.

The method 200 includes activating or otherwise triggering a kinematic sensing device (202) to cause the device to be awake and/or otherwise ready for sensing an impact, a blast event, or other event. The method also includes sensing and recording a signal (204) using the sensor or sensors 112. The method also includes receiving a first control signal prior to and/or during the event from a control sensor 128. (206) The method also includes receiving a second control signal during or after the event from the control sensor. (208) The method also includes comparing the first control signal to the second control signal. (210) Finally, the method also includes identifying the signal from the sensor 112 as a true positive signal. (212)

Activating or otherwise triggering a kinematic sensing device (202) may be performed in one or more ways. In one or more embodiments, activating or triggering a kinematic or motion sensing device may involve use of an active/sleep/wake mode. That is, for purposes of conserving battery power, the sensing device may remain asleep or in ultra-low-power mode, for example and then rapidly wake up into active mode to sense an impact. The waking up of the sensor may occur based on a sensed signal of interest, for example. In other embodiments, other systems or methods may be provided for triggering and/or awakening the sensing device.

In an alert or triggered state, the sensing device is ready for sensing an impact event, blast event, or other event. The method includes sensing and recording a signal. (204) In one or more embodiments, sensing and recording a signal may include sensing and recording signals across a range of variables including multiple linear accelerations and multiple angular accelerations. For example, sensing and recording a signal may include sensing linear accelerations along local x, y, and z axes of the sensing device. Still further, sensing and recording a signal may include sensing angular velocities about these local axes as well. The sensing may be performed with a sample rate consistent with kinematic sensing as discussed above. In the context of impact sensing, the signal may be analyzed by transferring the various components of the signal to a location of interest within the head, for example. The resulting accelerations and velocities may then be used to produce an assessment score or otherwise produce one or more metrics of the impact for use in monitoring potential injury or workload. In the context of blast sensing, the various components of the signal may be filtered to remove the effects of motion in lower frequency ranges and reveal the effects of a blast wave on the motion sensor.

In any of the above cases, but particularly in the case of impact sensing, the system may also identify the signals sensed by the sensors 112 as true positive signals. That is, in the context of impact sensing, which relates to motion of the human body, many other non-impact motions or actions taken with the sensors 112 may have a tendency to look like a bodily impact. Accordingly, it can be helpful to know if the sensors 112 are in position for sensing to help determine if a signal is a true positive signal. In the context of blast sensing, the frequencies sensed when the sensors 112 are exposed to blast event may be identifiable as blast events by filtering out frequencies more consistent with human body motion and, as such, being in position for sensing may not be quite as relevant. As mentioned, identifying the signals as true positive involves receiving first and second control signals (206/208). Receiving a first control signal includes receiving a signal from the control sensor 128. For example, in the case of a proximity sensor, a control signal may be received that provides a proximity reading. For example, a proximity reading ranging from approximately 400 units to approximately 2000 units or from approximately 500 units to approximately 1500, or from approximately 750 units to approximately 1000 units may be received. In one or more embodiments, the proximity reading may be a voltage measurement or another type of proximity measurement may be provided. However, as discussed in more detail below, the change in the reading may be used to assess the positional condition of the sensing system and, as such, the type of proximity sensor and the particular units may not be particularly important.

As mentioned, the control sensor reading is received before or during the impact event. That is, as will be discussed in more detail below, two readings may be compared at or around the time of the impact and, as such, a first reading is before the impact and/or during an early portion of the impact, while the second reading is during a later portion of the impact or after the impact. Receiving the second control signal includes receiving a signal from the control sensor 128. For example, in the case of a proximity sensor, a control signal may be received that provides a proximity reading. For example, a proximity reading ranging from approximately 400 units to approximately 2000 units or from approximately 500 units to approximately 1500, or from approximately 750 units to approximately 1000 units may be received. It is to be appreciated that while proximity sensors have been discussed as the control sensors 128, still other sensors may be used with different readings and the difference between first and second readings of those sensor may be used in the same way that the differences between the proximity readings is used. The particulars of the difference and/or comparison of the readings is discussed in more detail below.

The method also includes comparing the first control signal to the second control signal to determine if any appreciable change in the signal has occurred. (210) Where no appreciable change is present, the method identifies the sensed impact data as a true positive data set. However, where appreciable change is present in the proximity signals, the method may identify the sensed impact data as a false positive. That is, for example, where the proximity data suggests that the mouthguard is on the teeth prior to an impact and no real appreciable change in the proximity data occurs during an impact, then the data may be considered to be very likely relevant to an impact incurred by a user. In contrast, where the proximity data suggest a change in proximity at or around the time of impact, the data may not be relevant to an impact.

Some examples of false positive readings may be where, for example, a user is chewing on the mouthguard and when the user bites down, the proximity reading is relatively high because the sidewalls of the u-shaped portion of the mouthguard are pressed against one another. However, upon releasing the bite, the proximity sensor will show a low reading and this "impact" may be ruled out as a false positive. In contrast, when a user is playing a sport and actively has a mouthguard in place, the proximity reading will be relatively high since the mouthguard is on the teeth. Upon experiencing an impact, a well coupled mouthguard will remain in place and the proximity reading after the impact will also be relatively high. This may result in a small amount of difference between the before and after proximity readings and this impact may be identified as a true positive reading. It is to be appreciated that the "delta prox" (i.e., the difference between the before/after proximity readings) may not be the only metric for identifying true positives. That is, for example, when a mouthguard is in a gym bag and experiences an impact, the before/after proximity readings may be similar, but, nonetheless, neither reading is likely to show a high value indicating that the mouthguard is on the teeth or otherwise in position for sensing an impact. Accordingly, the proximity reading itself in addition to the delta prox may be used together to help identify true positive readings. Still further, multiple devices may be used as described in US Patent Application 16/682,767 filed on November 13, 2019. One example of multiple devices described in this application is a sensor in conjunction with a camera or video coverage of an event. Appendix A to this application includes test results relating to a sensor in conjunction with video footage of one or more events.

The comparison of the two control signals may be used to determine if any appreciable change has occurred. For example, appreciable change may include a change in the proximity reading or other control sensor reading that exceeds approximately 30%, or 25%, or 20%, or 15%, or 10%, or 5%, for example. In one or more embodiments, for example, a proximity reading of 0-50 units may suggest the sensing device is off the teeth while a reading of 500-1500 may suggest the sensing device is on the teeth. Where a sensing device has a bad fit or if a tooth is missing, for example, a proximity reading may be around 300 units. Where there is a good fit of a sensing device on the teeth, changes in the proximity reading may range from 0-25 units, for example (i.e., 0-5% change). However, where a sensing device has a bad fit or is wobbly, changes in the proximity reading may be above 5% and extend up to or above 20%, for example. Sensing devices going on or coming off the teeth may exhibit changes in a proximity reading of 100 to 1000 units, for example. Nonetheless, changes in the proximity reading ranging from 0-75 units (i.e., 0-15%) may be considered sufficient to indicate that the sensing device was on the teeth and remained there such that the impact is a true positive impact. Still other ranges may be used depending on calibration efforts and other factors.

In one or more embodiments, the sensors may be calibrated for each user, where the on-the-teeth proximity reading can be stored for each user such that a smaller or more refined change in the proximity reading may be used. In one or more embodiments, calibration may be performed by having the user indicate when the mouthguard is on teeth (e.g., by inputting this on a smartphone app where an input button is provided). The proximity reading may be stored in response to the user indication such that the "on teeth" proximity reading for that user is known. Variations from that reading may, thus, allow for identifying sensor readings and true or false positives. In one or more other embodiments, an auto-calibration may be performed where comparisons of multiple proximity readings are used to identify the "on teeth" reading for a particular user. Still other approaches to calibration may be provided. Where the control sensor is calibrated, a deviation from the on teeth reading ranging from 0-10% or 0-5% may be used to identify situations where the sensing system may not be suitably secured and, as such, a sensor reading may be identified as false positive, for example.

Where the difference between the proximity or other control sensor readings exceeds a threshold change (e.g., 5%, 10%, 15%), the signal from the sensor 112 may be discarded as a false positive. However, where the difference between the proximity or other control sensor readings does not exceed the threshold change, the signal from the sensor 112 may be identified as a true positive. In one or more embodiments, identifying a signal as true positive (212) may include attaching metadata, activating a display or a light, or more simply avoiding a process of discarding or ignoring the data. Still other approaches to identifying a signal as true positive may be provided.

As shown in FIG. 4, a method of workload monitoring (300) is also described. The workload monitoring may involve monitoring impacts and/or general motion of the sensors 112 and determining a workload of an athlete from those motions. That is, for example, while impact sensing mouthguards are commonly used to identify relatively high impacts (e.g., 15-100 g's), lower impacts (e.g., 2-15 g's) over a period of time may be used to identify workload, fatigue, or other conditions. On the one hand, this information may be useful when comparing athletes to one another based on workouts, work ethic, etc. On the other hand, this information may be useful for quantifying workloads during workouts such that fatigue or other metrics that may have a tendency or ability to lead to injury may be monitored.

The method 300 may include one or more of the steps of method 200, which may allow for the impacts sensed by the sensors 112 to be identified as true positive sensor results. This may be particularly advantageous in the context of workload monitoring because the impacts being sensed may be even more consistent with impacts experienced by the human body on a regular basis. As such, other efforts to rule out false positives (e.g., reviewing the waveforms generated by the impacts) may be less effective at these lower g forces because the waveforms may not be as distinguishable from other human motion in and of themselves. As such, having an ability to identify each of these lower g impacts as true positives for purposes of workload monitoring may be very valuable.

With true positive impact data in hand, the method 300 may include capturing impact data from sensors 112 on an ongoing basis. (302) In one or more embodiments, the method may include compiling the impact data (304) by, for example, determining a resultant head impact or collision force. In one or more embodiments, the several sensor signals may be used in their raw data form rather than computing a resultant. In either case, a resultant g-force or a series of g-forces may be captured and/or stored. In one or more embodiments, the impact data may be parsed into head impacts and collisions (306). For example, bumps may typically result in sensor accelerations less than or equal to 15 g's. Inertial forces on a user's body may also result in sensor accelerations less than or equal to 15 g's. Direct head impacts, on the other hand, may result in sensor accelerations that exceed 15 g's. In view of the above, one approach may be to categorize bumps and inertial forces on a user as being sensor accelerations from 0 g's up to and including 15 g's, while head impacts may be categorized as sensor accelerations exceeding 15 g's. In other embodiments, the cutoff between bumps/inertial forces and head impacts may be different and may be as low as, for example, 5-15 g's or as high as, for example, 15-25 g's. In one or more embodiments, the impact direction and location may also be relied on for assessing the nature of an impact. For example, the location and direction of the impact may be determined in a manner described in US Patent Application No.: 16/720,589 entitled Methods for Sensing and Analyzing Impacts and Performing an Assessment, and filed on December 19, 2019 with a priority date of December 19, 2018.

Having identified at least one way to distinguish between bumps/inertial forces and more severe head impacts, one or more methods for accumulating the impact data over time (308) may be provided. For example, sticking with g-forces and where resultants have been calculated, an accumulating resultant may be provided in the form of accumulated resultant g's experienced by the user. Where the data is used in its raw form, an accumulating g force along one or several axes may be accumulated. In this case, g's experienced by a user may be used as a sort of "proxy" for workload and relied on in comparison to other players and/or calibrated, so to speak, by becoming familiar with the amount of work is associated with a particular number of g's experienced. That is, player activity, tiredness, temperature, etc. may be gauged and begin to make the g's experienced more relevant. Moreover, another proxy for workload may, more simply, be the number of impacts experienced by a user where the number of impacts is stored, counted, or otherwise analyzed, for example. Still further, an actual energy-based workload may also be provided by calculating the energy experienced by the user. For example, kinetic energy calculations may be performed using known kinetic energy equations such as ½mv² + ½Iω² where 'm' is the mass of the player, 'v' is the velocity of the player calculated from the sensor information, 'I' is the moment of inertia of the player, and 'ω' is the angular velocity of the player. These calculated values may be accumulated over time to determine an actual workload of the player, for example.

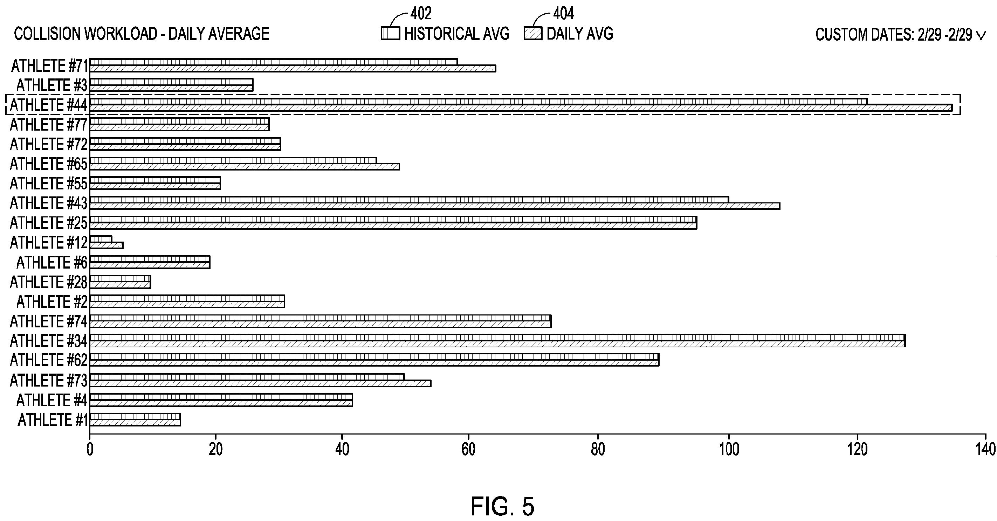

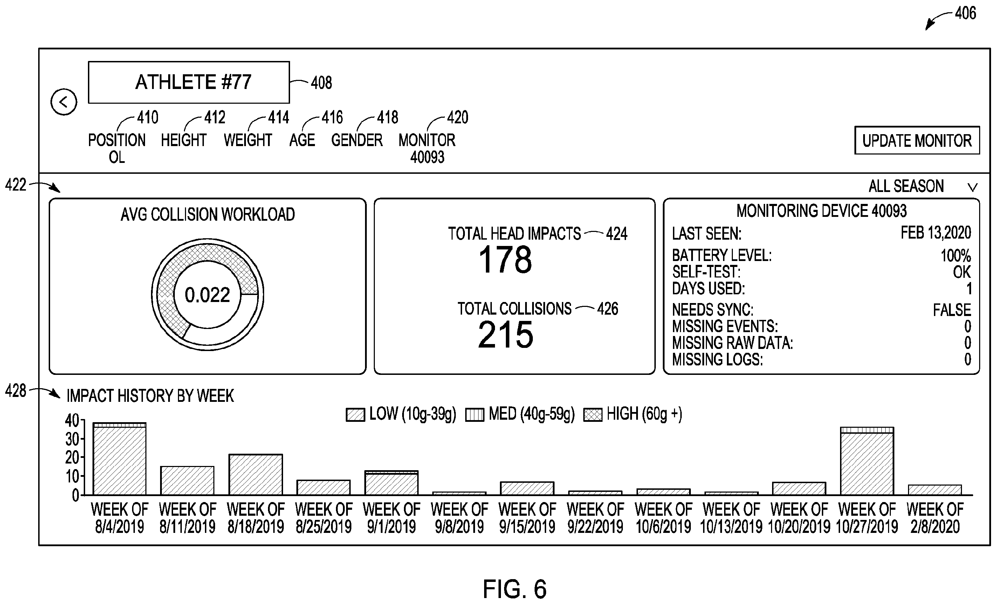

In one or more embodiments, the workload data may be collected or assessed over a time period. The time period selected may be over a season, a week, a practice session, or other logical time periods where periods of rest may intervene between periods of work. In one or more embodiments, average workloads may be calculated (310) based on the accumulating data. For example, as shown in FIG. 5, historical average workload 402 may include an average workload calculated based on historical accumulated data (e.g., g forces, impacts, or energy experienced) divided by the number of historical days covered. In addition, a daily average workload 404 may include an average workload calculated based on accumulated data (e.g., g forces, impacts, or energy experienced) from a particular day. As shown, the historical and daily averages may be attributed to particular athletes participating in a particular event, sport, season, etc. and they may tabulated or juxtaposed to allow for a quick view across several athletes as well as allowing for comparison therebetween. The method may also include presenting the data on a per user basis (312). For example, as shown in FIG. 6, a workload interface 406 may include an athlete identifier 408, a sports position 410, a height 412, a weight 414, an age 416, a gender 418, a monitor number 420 and/or other metrics to help identify the athlete and have an understanding of other relevant metrics at a glance. Still further the workload interface may include a report of average collision workload 422, which may be on a daily, weekly, or other timeframe basis. The workload interface 406 may also include a count of impacts such as a total number of impacts, which may be parsed into a total number of head impacts 424 and a total number of collisions 426. Still further information regarding the particular sensing device being used may be provided on the workload interface 406. In one or more embodiments, as shown in FIG. 6 a daily, weekly, monthly, or other time based graph 428 of workload may be provided to allow a user to see when particular levels of workload occurred or are occurring and allowing the user, coach, trainer, or other individual to recognize correlations between particular activities and workloads.

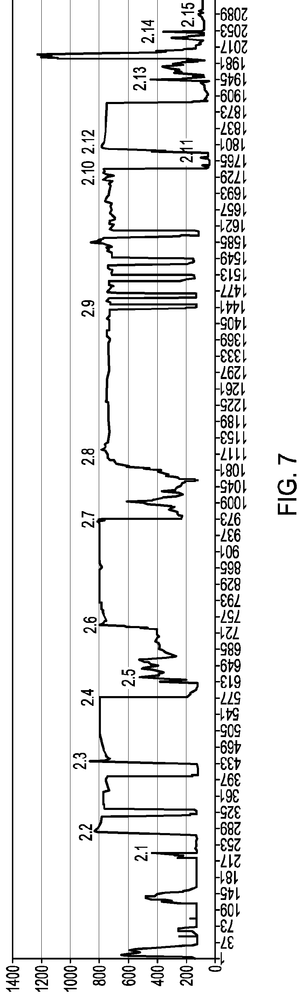

While proximity sensing has been discussed as being reviewed at particular times surrounding an impact, proximity sensing more continuously or periodically over time may also be used for purposes of identifying true positive impacts, for purposes of calibration, and/or for other purposes. As shown in FIG. 7, a series of events are reflected by the proximity sensors as follows:
2.1 Hold in Hand
2.2 Attempt to Activate with finger
2.3 Insert cleanly
2.4 Remove cleanly
2.5 Insert loosely into the mouth
2.6 On-the-teeth (Bite down)
2.7 Off the teeth but in-the-mouth
2.8 On-the-teeth (Bite down)
2.9 Fidget (off-on-off-repeat...)
2.10 Remove cleanly
2.11 Hold in hand
2.12 Attempt to activate with finger
2.13 Wash/Rinse
2.14 Place in charger case
2.15 Remove from charger case

In view of the above, timeframes such as at those extending between 2.3 and 2.4, between 2.6 and 2.7, and between 2.8 and 2.9, may be timeframes where true positive impact results may be collected. Moreover, these same timeframes may help to calibrate for this user that the on-the-teeth reading of the proximity sensor is approximately 800 units, for example, and readings that depart from 800 units may indicate that the sensing device is off of the teeth. Still other uses may be available based on the proximity data over time.

Study results based on video review of over 2000 detected true positive events found a true positive sensitivity of 98%, specificity of 99.5%, and positive predictive value of 96.6%, providing quantifiable trust that true positive events are being detected and reported with or without independent video verification.

For purposes of this disclosure, any system described herein may include any instrumentality or aggregate of instrumentalities operable to compute, calculate, determine, classify, process, transmit, receive, retrieve, originate, switch, store, display, communicate, manifest, detect, record, reproduce, handle, or utilize any form of information, intelligence, or data for business, scientific, control, or other purposes. For example, a system or any portion thereof may be a minicomputer, mainframe computer, personal computer (e.g., desktop or laptop), tablet computer, embedded computer, mobile device (e.g., personal digital assistant (PDA) or smart phone) or other hand-held computing device, server (e.g., blade server or rack server), a network storage device, or any other suitable device or combination of devices and may vary in size, shape, performance, functionality, and price. A system may include volatile memory (e.g., random access memory (RAM)), one or more processing resources such as a central processing unit (CPU) or hardware or software control logic, ROM, and/or other types of nonvolatile memory (e.g., EPROM, EEPROM, etc.). A basic input/output system (BIOS) can be stored in the non-volatile memory (e.g., ROM), and may include basic routines facilitating communication of data and signals between components within the system. The volatile memory may additionally include a high-speed RAM, such as static RAM for caching data.

Additional components of a system may include one or more disk drives or one or more mass storage devices, one or more network ports for communicating with external devices as well as various input and output (I/O) devices, such as digital and analog general purpose I/O, a keyboard, a mouse, touchscreen and/or a video display. Mass storage devices may include, but are not limited to, a hard disk drive, floppy disk drive, CD-ROM drive, smart drive, flash drive, or other types of non-volatile data storage, a plurality of storage devices, a storage subsystem, or any combination of storage devices. A storage interface may be provided for interfacing with mass storage devices, for example, a storage subsystem. The storage interface may include any suitable interface technology, such as EIDE, ATA, SATA, and IEEE 1394. A system may include what is referred to as a user interface for interacting with the system, which may generally include a display, mouse or other cursor control device, keyboard, button, touchpad, touch screen, stylus, remote control (such as an infrared remote control), microphone, camera, video recorder, gesture systems (e.g., eye movement, head movement, etc.), speaker, LED, light, joystick, game pad, switch, buzzer, bell, and/or other user input/output device for communicating with one or more users or for entering information into the system. These and other devices for interacting with the system may be connected to the system through I/O device interface(s) via a system bus, but can be connected by other interfaces such as a parallel port, IEEE 1394 serial port, a game port, a USB port, an IR interface, etc. Output devices may include any type of device for presenting information to a user, including but not limited to, a computer monitor, flat-screen display, or other visual display, a printer, and/or speakers or any other device for providing information in audio form, such as a telephone, a plurality of output devices, or any combination of output devices.

A system may also include one or more buses operable to transmit communications between the various hardware components. A system bus may be any of several types of bus structure that can further interconnect, for example, to a memory bus (with or without a memory controller) and/or a peripheral bus (e.g., PCI, PCIe, AGP, LPC, I2C, SPI, USB, etc.) using any of a variety of commercially available bus architectures.

One or more programs or applications, such as a web browser and/or other executable applications, may be stored in one or more of the system data storage devices. Generally, programs may include routines, methods, data structures, other software components, etc., that perform particular tasks or implement particular abstract data types. Programs or applications may be loaded in part or in whole into a main memory or processor during execution by the processor. One or more processors may execute applications or programs to run systems or methods of the present disclosure, or portions thereof, stored as executable programs or program code in the memory, or received from the Internet or other network. Any commercial or freeware web browser or other application capable of retrieving content from a network and displaying pages or screens may be used. In some embodiments, a customized application may be used to access, display, and update information. A user may interact with the system, programs, and data stored thereon or accessible thereto using any one or more of the input and output devices described above.

A system of the present disclosure can operate in a networked environment using logical connections via a wired and/or wireless communications subsystem to one or more networks and/or other computers. Other computers can include, but are not limited to, workstations, servers, routers, personal computers, microprocessor-based entertainment appliances, peer devices, or other common network nodes, and may generally include many or all of the elements described above. Logical connections may include wired and/or wireless connectivity to a local area network (LAN), a wide area network (WAN), hotspot, a global communications network, such as the Internet, and so on. The system may be operable to communicate with wired and/or wireless devices or other processing entities using, for example, radio technologies, such as the IEEE 802.xx family of standards, and includes at least Wi-Fi (wireless fidelity), WiMax, and Bluetooth wireless technologies. Communications can be made via a predefined structure as with a conventional network or via an ad hoc communication between at least two devices.

Hardware and software components of the present disclosure, as discussed herein, may be integral portions of a single computer, server, controller, or message sign, or may be connected parts of a computer network. The hardware and software components may be located within a single location or, in other embodiments, portions of the hardware and software components may be divided among a plurality of locations and connected directly or through a global computer information network, such as the Internet. Accordingly, aspects of the various embodiments of the present disclosure can be practiced in distributed computing environments where certain tasks are performed by remote processing devices that are linked through a communications network. In such a distributed computing environment, program modules may be located in local and/or remote storage and/or memory systems.

As will be appreciated by one of skill in the art, the various embodiments of the present disclosure may be embodied as a method (including, for example, a computer-implemented process, a business process, and/or any other process), apparatus (including, for example, a system, machine, device, computer program product, and/or the like), or a combination of the foregoing. Accordingly, embodiments of the present disclosure may take the form of an entirely hardware embodiment, an entirely software embodiment (including firmware, middleware, microcode, hardware description languages, etc.), or an embodiment combining software and hardware aspects. Furthermore, embodiments of the present disclosure may take the form of a computer program product on a computer-readable medium or computer-readable storage medium, having computer-executable program code embodied in the medium, that define processes or methods described herein. A processor or processors may perform the necessary tasks defined by the computer-executable program code. Computer-executable program code for carrying out operations of embodiments of the present disclosure may be written in an object oriented, scripted or unscripted programming language such as Java, Perl, PHP, Visual Basic, Smalltalk, C++, or the like. However, the computer program code for carrying out operations of embodiments of the present disclosure may also be written in conventional procedural programming languages, such as the C programming language or similar programming languages. A code segment may represent a procedure, a function, a subprogram, a program, a routine, a subroutine, a module, an object, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc. may be passed, forwarded, or transmitted via any suitable means including memory sharing, message passing, token passing, network transmission, etc.

In the context of this document, a computer readable medium may be any medium that can contain, store, communicate, or transport the program for use by or in connection with the systems disclosed herein. The computer-executable program code may be transmitted using any appropriate medium, including but not limited to the Internet, optical fiber cable, radio frequency (RF) signals or other wireless signals, or other mediums. The computer readable medium may be, for example but is not limited to, an electronic, magnetic, optical, electromagnetic, infrared, or semiconductor system, apparatus, or device. More specific examples of suitable computer readable medium include, but are not limited to, an electrical connection having one or more wires or a tangible storage medium such as a portable computer diskette, a hard disk, a random access memory (RAM), a read-only memory (ROM), an erasable programmable read-only memory (EPROM or Flash memory), a compact disc read-only memory (CD-ROM), or other optical or magnetic storage device. Computer-readable media includes, but is not to be confused with, computer-readable storage medium, which is intended to cover all physical, non-transitory, or similar embodiments of computer-readable media.

Various embodiments of the present disclosure may be described herein with reference to flowchart illustrations and/or block diagrams of methods, apparatus (systems), and computer program products. It is understood that each block of the flowchart illustrations and/or block diagrams, and/or combinations of blocks in the flowchart illustrations and/or block diagrams, can be implemented by computer-executable program code portions. These computer-executable program code portions may be provided to a processor of a general purpose computer, special purpose computer, or other programmable data processing apparatus to produce a particular machine, such that the code portions, which execute via the processor of the computer or other programmable data processing apparatus, create mechanisms for implementing the functions/acts specified in the flowchart and/or block diagram block or blocks. Alternatively, computer program implemented steps or acts may be combined with operator or human implemented steps or acts in order to carry out an embodiment of the invention.

Additionally, although a flowchart or block diagram may illustrate a method as comprising sequential steps or a process as having a particular order of operations, many of the steps or operations in the flowchart(s) or block diagram(s) illustrated herein can be performed in parallel or concurrently, and the flowchart(s) or block diagram(s) should be read in the context of the various embodiments of the present disclosure. In addition, the order of the method steps or process operations illustrated in a flowchart or block diagram may be rearranged for some embodiments. Similarly, a method or process illustrated in a flow chart or block diagram could have additional steps or operations not included therein or fewer steps or operations than those shown. Moreover, a method step may correspond to a method, a function, a procedure, a subroutine, a subprogram, etc.

As used herein, the terms "substantially" or "generally" refer to the complete or nearly complete extent or degree of an action, characteristic, property, state, structure, item, or result. For example, an object that is "substantially" or "generally" enclosed would mean that the object is either completely enclosed or nearly completely enclosed. The exact allowable degree of deviation from absolute completeness may in some cases depend on the specific context. However, generally speaking, the nearness of completion will be so as to have generally the same overall result as if absolute and total completion were obtained. The use of "substantially" or "generally" is equally applicable when used in a negative connotation to refer to the complete or near complete lack of an action, characteristic, property, state, structure, item, or result. For example, an element, combination, embodiment, or composition that is "substantially free of" or "generally free of" an element may still actually contain such element as long as there is generally no significant effect thereof.

Additionally, as used herein, the phrase "at least one of [X] and [Y]," where X and Y are different components that may be included in an embodiment of the present disclosure, means that the embodiment could include component X without component Y, the embodiment could include the component Y without component X, or the embodiment could include both components X and Y. Similarly, when used with respect to three or more components, such as "at least one of [X], [Y], and [Z]," the phrase means that the embodiment could include any one of the three or more components, any combination or sub-combination of any of the components, or all of the components.

In the foregoing description various embodiments of the present disclosure have been presented for the purpose of illustration and description. They are not intended to be exhaustive or to limit the invention to the precise form disclosed. Obvious modifications or variations are possible in light of the above teachings. The various embodiments were chosen and described to provide the best illustration of the principals of the disclosure and their practical application, and to enable one of ordinary skill in the art to utilize the various embodiments with various modifications as are suited to the particular use contemplated.

## Claims

1. A system for sensing true positive impacts to a user, the system comprising:
a sensing device (108) configured for secured coupling to a user and comprising:
a motion sensor (112) configured for sensing accelerations associated with an impact event and for generating an impact signal based on the impact event;
a control sensor (128) for sensing when the sensing device (108) is in position for sensing;
a computer-readable storage medium (114) having instructions stored thereon for:
receiving and capturing the impact signal from the motion sensor (112); and
comparing first and second control signals from the control sensor (128) to determine if the impact signal is a true positive signal, the first control signal from the control sensor being captured at a time prior to the impact event or during an early portion of the impact event and the second control signal from the control sensor being captured at a later portion of the impact event or after the impact event; and
a processor (116) for processing the instructions to capture the impact signal, perform the comparing, and identify the impact signal as a true positive signal indicative of an impact to the user based on the comparing.

2. The system of claim 1, wherein the control sensor (128) is a proximity sensor.

3. The system of claim 2, wherein comparing comprises comparing a proximity level of the first control signal to the proximity level of the second control signal.

4. The system of claim 3, wherein the processor (116) is configured to identify the impact signal as true positive when the second control signal differs from the first control signal by no more than 15%.

5. The system of claim 4, wherein the processor (116) is configured to identify the impact signal as true positive when the second control signal differs from the first control signal by no more than 5%.

6. The system of claim 4, wherein the processor (116) is further configured for comparing the first control signal from the control sensor (128) to a threshold value indicating that the sensing device (108) is in position for sensing.

7. The system of any one of claims 1 through 6, wherein the sensing device (108) is a mouthguard configured for secured coupling to teeth of a user.

8. The system of claim 7, wherein the control sensor (128) is arranged on the mouthguard for facing the teeth of the user.

9. A method of isolating true positive impacts from false positive impacts and storing the true positive impacts, the method comprising:
using a motion sensor (112) arranged on a sensing device (108) securely coupled to a user and configured for sensing accelerations and generating a signal of the accelerations, sensing an impact event and generating an impact signal;
using a control sensor (128) arranged on the sensing device (108):
sensing a first control signal prior to the impact event; and
sensing a second control signal after the impact event;
using a computer processor (116):
receiving the impact signal from the motion sensor (112);
receiving the first control signal from the control sensor (128);
receiving the second control signal from the control sensor (128);
comparing the first control signal to the second control signal;
identifying the impact signal as a true positive signal based on the comparing; and
discarding the impact signal if the impact signal is not identified as a true positive.

10. The method of claim 9, wherein the first and second control signals are proximity sensor signals.

11. The method of claim 10, wherein comparing comprises comparing a proximity level of the first control signal to the proximity level of the second control signal to identify a difference in proximity level.

12. The method of claim 11, wherein the difference in proximity level is further compared to a threshold.

13. The method of claim 12, wherein the threshold is met when the second control signal differs from the first control signal by less than 15%.

14. The method of claim 13, further comprising discarding the impact signal when the threshold is not met.

15. The method of any one of claims 9 through 14, comprising:
accumulating the true positive signals over time to establish a workload for the user; and
reporting the workload to the user.

## Patentansprüche

1. Ein System zum Erfassen richtig positiver Stöße auf einen Benutzer, wobei das System Folgendes beinhaltet:
eine Erfassungsvorrichtung (108), die zur sicheren Kopplung an einen Benutzer konfiguriert ist und Folgendes beinhaltet:
einen Bewegungssensor (112), der zum Erfassen von Beschleunigungen, die mit einem Stoßereignis assoziiert sind, und zum Erzeugen eines Stoßsignals basierend auf dem Stoßereignis konfiguriert ist;
einen Steuersensor (128) zum Erfassen, wenn sich die Erfassungsvorrichtung (108) in Position zum Erfassen befindet;
ein computerlesbares Speichermedium (114), das darauf gespeicherten Anweisungen für Folgendes aufweist:
Empfangen und Aufnehmen des Stoßsignals von dem Bewegungssensor (112); und
Vergleichen eines ersten und zweiten Steuersignals von dem Steuersensor (128), um zu bestimmen, ob das Stoßsignal ein richtig positives Signal ist, wobei das erste Steuersignal von dem Steuersensor zu einem Zeitpunkt vor dem Stoßereignis oder während eines frühen Abschnitts des Stoßereignisses aufgenommen wird und das zweite Steuersignal von dem Steuersensor zu einem späteren Abschnitt des Stoßereignisses oder nach dem Stoßereignis aufgenommen wird; und
einen Prozessor (116) zum Verarbeiten der Anweisungen, das Stoßsignal aufzunehmen, den Vergleich durchzuführen und basierend auf dem Vergleich das Stoßsignal als ein richtig positives Signal zu identifizieren, das einen Stoß auf den Benutzer anzeigt.

2. System gemäß Anspruch 1, wobei der Steuersensor (128) ein Näherungssensor ist.

3. System gemäß Anspruch 2, wobei das Vergleichen das Vergleichen eines Näherungspegels des ersten Steuersignals mit dem Näherungspegel des zweiten Steuersignals beinhaltet.

4. System gemäß Anspruch 3, wobei der Prozessor (116) konfiguriert ist, um das Stoßsignal als richtig positiv zu identifizieren, wenn sich das zweite Steuersignal um nicht mehr als 15 % von dem ersten Steuersignal unterscheidet.

5. System gemäß Anspruch 4, wobei der Prozessor (116) konfiguriert ist, um das Stoßsignal als richtig positiv zu identifizieren, wenn sich das zweite Steuersignal um nicht mehr als 5 % von dem ersten Steuersignal unterscheidet.

6. System gemäß Anspruch 4, wobei der Prozessor (116) ferner zum Vergleichen des ersten Steuersignals von dem Steuersensor (128) mit einem Schwellenwert, der anzeigt, dass sich die Erfassungsvorrichtung (108) in Position zum Erfassen befindet, konfiguriert ist.

7. System gemäß einem der Patentansprüche 1 bis 6, wobei die Erfassungsvorrichtung (108) ein Mundschutz ist, der zur sicheren Kopplung an Zähne eines Benutzers konfiguriert ist.

8. System gemäß Anspruch 7, wobei der Steuersensor (128) auf dem Mundschutz angeordnet ist, um den Zähnen eines Benutzers zugewandt zu sein.

9. Ein Verfahren zum Isolieren richtig positiver Stöße von falsch positiven Stößen und zum Speichern der richtig positiven Stöße, wobei das Verfahren Folgendes beinhaltet:
unter Verwendung eines Bewegungssensors (112), der auf einer Erfassungsvorrichtung (108) angeordnet ist, die sicher an einen Benutzer gekoppelt ist, und der zum Erfassen von Beschleunigungen und zum Erzeugen eines Signals der Beschleunigungen konfiguriert ist, Erfassen eines Stoßereignisses und Erzeugen eines Stoßsignals;
unter Verwendung eines Steuersensors (128), der auf der Erfassungsvorrichtung (108) angeordnet ist:
Erfassen eines ersten Steuersignals vor dem Stoßereignis; und
Erfassen eines zweiten Steuersignals nach dem Stoßereignis;
unter Verwendung eines Computerprozessors (116):
Empfangen des Stoßsignals von dem Bewegungssensor (112);
Empfangen des ersten Steuersignals von dem Steuersensor (128);
Empfangen des zweiten Steuersignals von dem Steuersensor (128);
Vergleichen des ersten Steuersignals mit dem zweiten Steuersignal;
Identifizieren des Stoßsignals als ein richtig positives Signal basierend auf dem Vergleich; und
Verwerfen des Stoßsignals, falls das Stoßsignal nicht als richtig positiv identifiziert wird.

10. Verfahren gemäß Anspruch 9, wobei das erste und das zweite Steuersignal Näherungssensorsignale sind.

11. Verfahren gemäß Anspruch 10, wobei das Vergleichen das Vergleichen eines Näherungspegels des ersten Steuersignals mit dem Näherungspegel des zweiten Steuersignals beinhaltet, um eine Differenz in dem Näherungspegel zu identifizieren.

12. Verfahren gemäß Anspruch 11, wobei die Differenz in dem Näherungspegel ferner mit einem Schwellenwert verglichen wird.

13. Verfahren gemäß Anspruch 12, wobei der Schwellenwert erfüllt wird, wenn sich das zweite Steuersignal um weniger als 15 % von dem ersten Steuersignal unterscheidet.

14. Verfahren gemäß Anspruch 13, das ferner das Verwerfen des Stoßsignals beinhaltet, wenn der Schwellenwert nicht erfüllt wird.

15. Verfahren gemäß einem der Ansprüche 9 bis 14, das Folgendes beinhaltet:
Akkumulieren der richtig positiven Signale über die Zeit, um eine Arbeitslast für den Benutzer zu erstellen; und
Melden der Arbeitslast an den Benutzer.

## Revendications

1. Un système pour capter des impacts de vrais positifs sur un utilisateur, le système comprenant :
un dispositif capteur (108) configuré pour un couplage sécurisé sur un utilisateur et comprenant :
un capteur de mouvement (112) configuré pour capter des accélérations associées à un événement d'impact et pour générer un signal d'impact basé sur l'événement d'impact ;
un capteur de commande (128) pour capter quand le dispositif capteur (108) est en position pour capter ;
un support de stockage lisible par ordinateur (114) sur lequel sont stockées des instructions pour :
recevoir et capturer le signal d'impact provenant du capteur de mouvement (112) ; et
comparer des premier et deuxième signaux de commande provenant du capteur de commande (128) afin de déterminer si le signal d'impact est un signal de vrai positif, le premier signal de commande provenant du capteur de commande étant capturé à un moment avant l'événement d'impact ou pendant une portion précoce de l'événement d'impact et le deuxième signal de commande provenant du capteur de commande étant capturé à une portion ultérieure de l'événement d'impact ou après l'événement d'impact ; et
un processeur (116) pour traiter les instructions afin de capturer le signal d'impact, effectuer la comparaison, et identifier le signal d'impact comme un signal de vrai positif indicatif d'un impact sur l'utilisateur sur la base de la comparaison.

2. Le système de la revendication 1, dans lequel le capteur de commande (128) est un capteur de proximité.

3. Le système de la revendication 2, dans lequel la comparaison comprend le fait de comparer un niveau de proximité du premier signal de commande au niveau de proximité du deuxième signal de commande.

4. Le système de la revendication 3, dans lequel le processeur (116) est configuré afin d'identifier le signal d'impact comme un vrai positif lorsque le deuxième signal de commande diffère du premier signal de commande de pas plus de 15 %.

5. Le système de la revendication 4, dans lequel le processeur (116) est configuré afin d'identifier le signal d'impact comme un vrai positif lorsque le deuxième signal de commande diffère du premier signal de commande de pas plus de 5 %.

6. Le système de la revendication 4, dans lequel le processeur (116) est en outre configuré pour comparer le premier signal de commande provenant du capteur de commande (128) à une valeur de seuil indiquant que le dispositif capteur (108) est en position pour capter.

7. Le système de l'une quelconque des revendications 1 à 6, dans lequel le dispositif capteur (108) est un protège-dents configuré pour un couplage sécurisé sur les dents d'un utilisateur.

8. Le système de la revendication 7, dans lequel le capteur de commande (128) est disposé sur le protège-dents pour faire face aux dents de l'utilisateur.

9. Un procédé pour isoler des impacts de vrais positifs d'impacts de faux positifs et stocker les impacts de vrais positifs, le procédé comprenant :
à l'aide d'un capteur de mouvement (112) disposé sur un dispositif capteur (108) couplé de manière sécurisée sur un utilisateur et configuré pour capter des accélérations et générer un signal des accélérations, le fait de capter un événement d'impact et de générer un signal d'impact ;
à l'aide d'un capteur de commande (128) disposé sur le dispositif capteur (108) :
le fait de capter un premier signal de commande avant l'événement d'impact ; et
le fait de capter un deuxième signal de commande après l'événement d'impact ;
à l'aide d'un processeur informatique (116) :
le fait de recevoir le signal d'impact provenant du capteur de mouvement (112) ;
le fait de recevoir le premier signal de commande provenant du capteur de commande (128) ;
le fait de recevoir le deuxième signal de commande provenant du capteur de commande (128) ;
le fait de comparer le premier signal de commande au deuxième signal de commande ;
le fait d'identifier le signal d'impact comme un signal de vrai positif sur la base de la comparaison ; et
le fait de rejeter le signal d'impact si le signal d'impact n'est pas identifié comme un vrai positif.

10. Le procédé de la revendication 9, dans lequel les premier et deuxième signaux de commande sont des signaux de capteur de proximité.

11. Le procédé de la revendication 10, dans lequel la comparaison comprend le fait de comparer un niveau de proximité du premier signal de commande au niveau de proximité du deuxième signal de commande afin d'identifier une différence de niveau de proximité.

12. Le procédé de la revendication 11, dans lequel la différence de niveau de proximité est en outre comparée à un seuil.

13. Le procédé de la revendication 12, dans lequel le seuil est atteint lorsque le deuxième signal de commande diffère du premier signal de commande de moins de 15 %.

14. Le procédé de la revendication 13, comprenant en outre le fait de rejeter le signal d'impact lorsque le seuil n'est pas atteint.

15. Le procédé de l'une quelconque des revendications 9 à 14, comprenant :
le fait d'accumuler les signaux de vrais positifs au fil du temps afin d'établir une charge de travail pour l'utilisateur ; et
le fait de rapporter la charge de travail à l'utilisateur.
